# FASCICULE DE BREVET EUROPEEN

(11) **EP 3 295 909 B1**
(45) Date de publication et mention de la délivrance du brevet: **02.06.2021**
(21) Numéro de dépôt: 17183058.1
(22) Date de dépôt: 25.07.2017
(51) Int. Cl.: A61F 13/02, A61F 13/00

(54) **PANSEMENT POUR POINT DE PONCTION OU D'INFUSION, EN PARTICULIER POUR HEMODIALYSE**
VERBANDMATERIAL FÜR PUNKTIONS- ODER INFUSIONSSTELLE, INSBESONDERE FÜR DIE HÄMODIALYSE
DRESSING FOR A PUNCTURE OR INFUSION SITE, IN PARTICULAR FOR HAEMODIALYSIS

(30) Priorité: 14.09.2016 FR 1658547
(43) Date de publication de la demande: 21.03.2018
(73) Titulaire: Nephrokit, 78110 Le Vesinet (FR)
(72) Inventeur: CHAWKI, Mokhtar, 78110 LE VESINET (FR)
(74) Mandataire: Demulsant, Xavier

(56) Documents cités:
- WO-A2-94/21207
- FR-A1- 2 893 249
- US-A1- 2008 132 821

## Description

L'invention se rapporte au domaine médical ou vétérinaire.

L'invention concerne plus particulièrement un pansement d'un point de ponction ou d'un point d'infusion.

Par « ponction », on désigne ici l'opération par laquelle une substance, généralement liquide, est évacuée d'une partie du corps d'un homme ou d'un animal.

Un exemple de ponction est le prélèvement sanguin, soin courant permettant des examens de laboratoire sur un échantillon de sang prélevé par ponction veineuse, capillaire ou artérielle. L'aiguille hypodermique utilisée pour le prélèvement sanguin est souvent reliée à des tubes sous vide (par exemple Vacutainer®, commercialisés par la société Becton Dickinson).

Par « infusion », on désigne ici l'opération par laquelle une substance, généralement liquide, est introduite dans une cavité ou dans les vaisseaux du corps d'un homme ou d'un animal.

Un exemple d'infusion est la perfusion intraveineuse, technique courante d'infusion parentérale permettant l'administration en goutte à goutte de médicaments, de liquides ou produits sanguins dans les veines, généralement une veine périphérique du membre supérieur. La perfusion intraveineuse permet notamment l'administration de solutés de correction de la volémie.

Par « point de ponction ou point d'infusion », on désigne ici une zone de peau du corps d'un homme ou d'un animal au travers de laquelle passe un dispositif de ponction ou d'infusion (en particulier une aiguille, un cathéter, une canule).

Par « pansement d'un point de ponction ou d'infusion » on désigne ici un dispositif de protection recouvrant la plaie formée par le point de ponction ou d'infusion.

L'invention se rapporte plus particulièrement à l'utilisation médicale ou vétérinaire d'un point de ponction ou d'infusion vasculaire, par exemple perfusions à aiguilles/cathéters ou dispositifs intraveineux courts de type cathlon® (canules en PTFE).

L'invention se rapporte plus particulièrement encore au domaine de l'hémodialyse.

Il est courant de retirer ou d'injecter des fluides ou d'administrer des médicaments à un patient ou un animal, par l'intermédiaire d'un tube fixé à une aiguille ou un cathéter. Il est estimé qu'environ 80 % des patients hospitalisés font l'objet d'un traitement administré par cathéter intraveineux.

Pour la ponction ou l'infusion, un dispositif est inséré au travers de la peau (aiguille hypodermique, cathéter, canule).

Par exemple, les aiguilles à ailettes sont employées pour les transfusions, les prises de sang notamment. Ces aiguilles tirent leur nom de ce qu'elles sont pourvues d'une zone de pincement manuel en forme d'ailes de papillon. Le repli de ces ailettes l'une contre l'autre permet une bonne préhension et facilite l'insertion de l'aiguille sous la peau. Une fois l'aiguille mise en place, les ailettes viennent classiquement se reposer sur la peau et une bande adhésive est placée sur ces ailettes et sur la peau en vue d'éviter l'extraction de l'aiguille. Des exemples d'aiguilles à ailettes peuvent être trouvés dans les brevets américains délivrés sous les numéros suivants: 2725058, 3064648, 3640275, 4194504, 4300553, 4627842, 5108376, 5149328, 6270480.

Pour certains patients, les cathéters périphériques sont d'usage chronique. C'est le cas notamment des malades souffrant d'insuffisance rénale aiguë ou chronique et traités par hémodialyse, ou épuration extra rénale.

Trois types d'accès vasculaires prédominent pour l'hémodialyse: les fistules artérioveineuses FAV, les prothèses artérioveineuses ou grafts, et les cathéters veineux centraux CVC.

Les FAV sont des anastomoses créées chirurgicalement pour connecter une artère et une veine du patient, couramment dans l'avant-bras, ou le bras, le plus souvent entre une artère radiale ou humérale et sa veine homonyme. Cette anastomose permet d'augmenter le débit sanguin au sein de ladite veine.

L'aiguille amenant le sang du patient vers l'appareil de dialyse est appelée artère, celle restituant le sang vers le patient étant appelée veine.

Lorsque l'aiguille est retirée d'un vaisseau sanguin ou d'une fistule de dialyse, plusieurs risques sont à prendre en compte : risque d'hémorragie, risque de projection de sang, risque de contamination du personnel soignant, risque d'hématome, risque de contamination bactérienne.

Le risque hémorragique est très important pour certains malades souffrant d'insuffisance rénale aiguë ou chronique et traités par hémodialyse. Lors de l'hémodialyse, un anticoagulant est en effet souvent employé pour limiter les risques de colmatage des lumières des capillaires par thrombose. A la fin de la séance de dialyse, toute hémorragie à partir du point de ponction peut s'avérer fatale pour le patient.

Les risques de projection de sang et de contamination sont importants, l'expansion du SIDA et des hépatites, entre autres maladies contagieuses par voie sanguine, n'ayant fait que rendre ce risque plus redouté. Selon la circulaire de la Délégation générale de la santé, datée du 20 avril 1998 (accessible à l'adresse http://www.sante.gouv.fr), relative à la prévention de la transmission d'agents infectieux véhiculés par le sang ou les liquides biologiques lors des soins dans les établissements de santé, un accident d'exposition au sang (AES) est défini comme « *tout contact avec du sang ou un liquide biologique contenant du sang et comportant soit une effraction cutanée soit une projection sur une muqueuse (œil) ou sur une peau lésée* ». Le risque de transmission de maladies infectieuses lors d'un AES en hémodialyse est actuellement dominé par le virus de l'hépatite C, en raison de sa prévalence chez les patients hémodialysés. Le risque de transmission du VIH après exposition au sang d'un patient porteur du VIH est estimé en moyenne à 0.32%. Le risque de transmission du VHB à partir d'un patient infecté est très élevé : entre 2% et 40%. Cette forte contagiosité est liée à la quantité très importante de virus présents dans le sang et les liquides biologiques (entre 1 million à 1 milliard de particules virales par ml). Selon le rapport RAISIN (surveillance des accidents d'exposition au sang dans les établissements de santé Français en 2005, disponible à l'adresse http://www.invs.sante.fr), la majorité des accidents d'exposition du sang surviennent au moment du retrait d'une aiguille à travers la peau. Selon le rapport du réseau AES-Raisin, pour les années 2013-2014, le taux d'AES pour 100 lits est de 6 % environ, et les AES résultent dans 20% des cas d'une projection, notamment lors du retrait d'une aiguille à travers la peau d'un patient.

Pour les séances d'hémodialyse sur FAV, le moment le plus propice au risque de contamination par projection de sang est celui de la compression, principalement au moment du retrait de l'aiguille. En effet, la compresse hémostatique peut être imparfaitement positionnée sur le point de ponction, en particulier pour des FAV présentant un fort débit ou des zones anévrysmales.

En fin de séance de dialyse, lors du débranchement, il existe un risque de contamination bactérienne, généralement staphylococcique. Ce risque est élevé au moment de la compression des points de ponction ou lors d'une récidive du saignement à distance de la séance de dialyse.

La plaie formée lors de chaque séance de dialyse présente des caractéristiques spécifiques.

En premier lieu, cette plaie intervient dans une zone de faible étendue, de manière répétée, avec des aiguilles de gros diamètre. Une séance d'hémodialyse dure environ quatre heures, et doit être effectuée trois fois par semaine. Les aiguilles utilisées pour ponctionner les FAV sont de gros calibre, d'un diamètre interne variant typiquement entre 1.6 et 2 mm.

En deuxième lieu, la peau entourant le site de ponction ou d'infusion dans la FAV est en principe non exsudative, et non secrétante, seul du sang pouvant être présent, le saignement post dialyse étant une complication connue. Certains patients restent à risque, même après compression manuelle de trente minutes (Perera et al A novel use of 2 octyl-cyanocrylate controlling post hemodialysis site hemorrhage, The Journal of Emergency Medecine, 44 vol 2, pp. 467-468 2013). L'hémostase est obtenue dans un délai variable : de l'ordre de 15 à 20 minutes, selon Schwab et al, Prevention of hemodialysis fistula thrombosis. Early detection of venous stenosis, Kidney International, 36, pp. 707-711 (1989*)*, et pourrait être réduite à 3 minutes environ par l'administration locale de thrombine selon Varizi, Topical thrombine and control of bleeding from the fistula puncture sites in dialyzed patients, Nephron 24, pp. 254-256 (1979*).* L'emploi de poly-β-1→4-N-acetylglucosamine permettrait des temps de compression de l'ordre de une à quatorze minutes (US 8992453, page 4 lignes 44-54). Chez les dialysés porteurs d'une FAV à fort débit sanguin, l'effet des anticoagulants est tel que le temps de saignement, au point de ponction, peut valoir jusqu'à trente minutes (voir WO 2004/060245 page 1, 3^{ème} paragraphe) voire quarante-cinq minutes, après la séance de dialyse (voir WO 03/099143, page 1 lignes 11-20). Il existe un grand nombre de brevets et demandes de brevet pour des pansements compressifs, pour tenter d'apporter une solution à ces temps de saignements (Voir par exemple US2004/0092999, WO99/08723, US2005/0256438, WO2007/044647, WO03/099143, US5891074, US20060155235, US 3490448, US 6316686).

En troisième lieu, la plaie intervient dans une zone déformée. La création d'une FAV modifie en effet fortement l'aspect de l'avant-bras du patient, par création de zones anévrysmales.

Il est par ailleurs fréquent que les patients dialysés soient âgés, avec les conséquences qui en résultent pour la sensibilité de la peau aux pansements.

Les moyens mis en oeuvre dans les unités d'hémodialyse pour limiter les risques d'AES et d'hémorragie ne sont pas standardisés.

Les protocoles de débranchement ne sont en particulier pas uniformes, plusieurs paramètres étant pris en compte : autonomie des patients, capacité physique des patients leur permettant plus ou moins de s'impliquer dans leur prise en charge, fragilité de la peau de patients pour la plupart âgés, existence ou non de zones anévrysmales, prise de médicaments anticoagulants de type acide acétylsalicylique ou Anti Vitamine K ou anti-inflammatoires.

Le débit de la FAV, ainsi que la présence d'hyperpression par sténose de l'abord vasculaire peuvent concourir à prolonger le temps de compression.

On connaît, dans l'art antérieur, plusieurs techniques visant à éviter les risques d'AES et d'hémorragie qui viennent d'être décrits.

Les moyens permettant d'obtenir l'hémostase sur une FAV peuvent être classés en quatre catégories :
- compression manuelle,
- dispositifs mécaniques comprenant un mécanisme à ressort (fistula pressure clamp), voir par exemple CN203169258,
- bandes assurant une compression par serrage,
- pads comprenant une compresse chargée d'un agent hémostatique (gélatine, collagène, chitosane, oxyde de cellulose, kaolin, zéolite), voir par exemple Bachtell et al, Treatment of dialysis access puncture wound bleeding with chitosan dressings, Dialysis and transplantation, 2006 pp. 1-6*.*

Selon une technique extrêmement courante permettant d'obtenir l'hémostase sur une FAV, une compresse, un tampon ou du coton est placé au point de ponction, et une pression manuelle est appliquée sur cette compresse ou ce coton, une ou plusieurs bandes adhésives maintenant en place la compresse ou le coton après relâchement de la pression manuelle.

Le demandeur a constaté que cette technique courante pose plusieurs difficultés.

Chez les dialysés porteurs d'une FAV à fort débit sanguin, l'effet des anticoagulants est tel que le temps de saignement au point de ponction peut valoir jusqu'à quarante-cinq minutes, après la séance de dialyse (voir document WO 03/099143 page 1 lignes 11 à 20). Le maintien d'une pression sur la FAV pendant un temps aussi long est fastidieux et fatiguant. Les personnes souffrant de maladies neurodégénératives (Parkinson, syndrome parkinsonien, Alzheimer) peuvent ne pas être en mesure de maintenir une pression manuelle au point de ponction. Il en va de même chez les patients agités, ou dépressifs, épileptiques, ou atteints de chorée, ou frappés de convulsions pour diverses raisons.

Il est nécessaire de contrôler régulièrement l'arrêt du saignement au point de ponction ou d'infusion, ce qui nécessite d'enlever la compresse ou le coton. Ce contrôle peut provoquer une projection de sang, avec risque de contamination du personnel soignant, ou bien encore une hémorragie, en particulier lorsque le patient est agité ou pour les patients dialysés devant comprimer à la fois l'accès veineux et l'accès artériel de la FAV. Un saignement peut survenir lors de la compression du point de ponction, obligeant à changer de compresse, la présence de sang occultant le point de ponction. La vérification régulière de l'arrêt du saignement par retrait partiel ou total de la compresse peut entraîner l'adhésion de la compresse hémostatique ou non sur le site de ponction, avec risque de décollement du clou plaquettaire.

Le document US 2004/0092999 décrit un bracelet élastique en latex sur lequel est collée une pièce hémisphérique en gomme ou latex, ce bracelet pouvant servir de garrot avant ponction veineuse, et facilitant l'hémostase après ponction, la pièce de gomme venant comprimer le point de ponction au travers d'une compresse. La pièce de gomme décrite dans ce document antérieur permet d'éviter l'application d'une pression manuelle au point de ponction. Mais l'utilisation de ce bracelet ne permet pas d'éviter les risques de projection de sang ou d'hémorragie, lorsque l'on enlève la pièce de compression pour contrôler le point de ponction. Les pansements décrits dans les documents WO 99/08723, US 2005/0256438 présentent les mêmes inconvénients. Il en va de même du pansement compressif pour FAV décrit dans le document WO 03/099143.

Le document US 5891074 décrit un pansement compressif comprenant une mousse de polymère absorbante placée en regard du point de ponction ou d'infusion. La mousse de polymère absorbante est par exemple une mousse de polyuréthane commercialisée par la société Avitar sous la marque Hydrasorb®. En variante, une pièce en acier à ressort ou en matériau polymère tel que polycarbonate, polyéthylène, polyuréthane est placée en contact direct avec la peau. Le pansement décrit dans le document US 5891074, comme un très grand nombre de pansements proposés dans l'art antérieur, vise l'application automatique d'une pression au lieu de ponction ou d'infusion, en lieu et place d'une application manuelle.

Au-delà des agents hémostatiques cités dans le document US 2006/0155235, on connaît dans l'art antérieur un grand nombre d'agents chimiques favorisant l'hémostase (à base de gélatine, de collagène, d'oxyde de cellulose, de chitosane). Bachtell et al (Treatment of dialysis access puncture wound bleeding with chitosan dressings, Dialysis & Transplantation, Novembre 2006) décrivent l'utilisation d'un pansement hémostatique commercialisé par la société HemCon, ce pansement permettant de réduire à quelques minutes le temps de compression sur les FAV pour obtenir l'hémostase. Les pansements proposés par la société HenCon ont été largement utilisés dans des contextes d'urgence. Ces pansements, par exemple commercialisés sous l'appellation Hencon Chito-Flex ® contiennent des agents hémostatiques (dérivé de chitosane, voir http://www.fda.gov). Ces pansements présentent l'inconvénient d'occulter le point de ponction que l'on ne peut surveiller visuellement tout en comprimant le point de saignement cutané.

Le présent inventeur a développé un pansement pour fistule artérioveineuse permettant de réduire significativement le temps de saignement en fin de dialyse (voir Boulanger et al, Evaluation of post-puncture bleeding time of arteriorvenous fistulas with Iris® bandage, J Vasc Access 2014 pp. 102-107). Ce pansement est commercialisé sous la marque Iris et permet de réduire le temps de compression post dialyse des fistules artério veineuses.

Le présent inventeur a constaté que certains patients présentaient toujours des temps des saignements post dialyse prolongés, en dépit de l'utilisation des meilleurs techniques disponibles. Le saignement post dialyse prolongé est une complication fréquente et sévère, qui altère la qualité de vie des patients et allonge la durée des séances de dialyse, le personnel médical étant particulièrement sollicité en fin de séance de dialyse.

L'invention vise à apporter une solution aux problèmes présentés ci-dessus.

L'invention vise notamment à proposer un dispositif permettant de panser un point de ponction ou d'infusion, ce dispositif ne présentant pas les inconvénients de ceux connus antérieurement, et assurant une protection élevée contre les AES, les hémorragies et les contaminations bactériennes, en particulier mais non exclusivement pour les abords vasculaires de dialyse.

A ces fins, il est proposé, selon un premier aspect, un pansement pour point de ponction ou d'infusion, comprenant une feuille en matériau polymère micro-perforée, une première face de cette feuille étant recouverte d'un adhésif sensible à la pression, la face opposée de cette feuille étant non adhésive, le pansement comprenant une bande de matériau, fixée sur la face non adhésive de la feuille de matériau polymère, cette bande de matériau étant compressible et imperméable. La feuille en matériau polymère micro-perforée est pourvue de perforations d'un diamètre inférieur à 0.5 mm.

Lors du mouvement de retrait d'une aiguille d'un point de ponction ou d'infusion, en particulier en fin de dialyse, l'application de la feuille adhésive sur ce point de ponction ou d'infusion et autour de ce point peut conduire à la formation d'un passage direct d'air, entre la feuille adhésive et la peau du patient, ce passage direct étant l'empreinte du volume occupé par l'aiguille. L'application d'une pression manuelle sur la bande de matériau compressible et imperméable permet de refermer ce passage direct, et permet d'empêcher la création d'une dépression dans ce passage, lors du retrait complet de l'aiguille.

Avantageusement, la bande de matériau compressible et imperméable forme un cadre. Cette disposition permet d'employer le pansement dans diverses orientations.

Selon diverses mises en oeuvre, le pansement présente les caractères suivants, le cas échéant combinés :
- la bande de matériau compressible et imperméable est en polymère expansé, ou en matériau non-tissé à base de cellulose, ou à base de polyéthylène vinyl acétate ;
- la bande de matériau compressible et imperméable est en mousse de polyéthylène ;
- la feuille en matériau polymère micro-perforée est transparente, translucide ou semi-transparente ;
- la feuille en matériau polymère micro-perforée est en polyéthylène pourvue d'un adhésif acrylique ;
- la densité de perforations de la feuille en matériau polymère micro-perforée est de l'ordre de cent par centimètre carré.

Il est proposé, selon un deuxième aspect, un kit de perfusion, de drainage, ou de cathétérisme, comprenant un pansement tel que présenté ci-dessus, et une aiguille ou une canule ou un cathéter ou un drain.

Il est proposé, l'utilisation, en tant que pansement de fistule artério-veineuse FAV, d'un pansement tel que présenté ci-dessus.

D'autres objets et avantages de l'invention apparaîtront à la lumière de la description suivante de modes de réalisation, description qui va être effectuée en se référant aux dessins annexés, dans lesquels :
- la figure 1 est une vue en perspective d'un avant-bras de patient, pourvu d'une FAV, une aiguille à ailettes étant en place dans cette FAV ;
- la figure 2 est une vue analogue à la figure 1, montrant une première étape du procédé ;
- la figure 3 est une vue analogue aux figures 1 et 2, montrant une deuxième étape du procédé
- la figure 4 est une vue en plan d'un pansement, selon un mode de réalisation ;
- la figure 5 est une vue latérale du pansement de la figure 4.

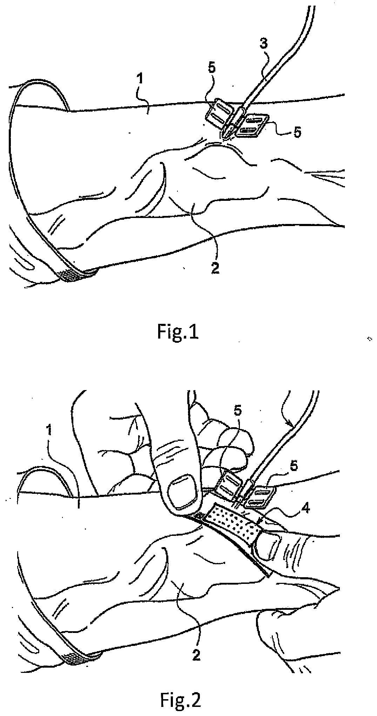

On se reporte aux figures 1 à 3.

Sur la figure 1 est représenté l'avant-bras 1 d'un patient, porteur d'une FAV. A fins de simplification, une seule aiguille est encore en place dans cette FAV. Ainsi que le comprendra l'homme du métier, la procédure décrite en référence aux figures s'applique successivement aussi bien à l'aiguille de la voie veineuse qu'à l'aiguille de la voie artérielle.

Sur les figures annexées, l'aiguille est du type aiguille à ailettes. Il est entendu toutefois que la procédure décrite concerne également les aiguilles dépourvues d'ailettes, ou bien encore les cathéters (par exemple de type cathlon®) ou bien encore les canules.

La FAV, abord vasculaire de première indication et le plus répandu pour le patient dialysé, entraîne l'apparition de zones anévrismales 2, visibles sur les figures, la peau étant très déformée au voisinage de la FAV. Les FAV sont souvent de petite longueur, de sorte que les deux aiguilles artère et veine doivent être placées proches l'une de l'autre. L'utilisation de bandes de fixation peut provoquer une irritation répétée de la peau, favorisant les excoriations particulièrement pourvoyeuses de contamination bactérienne. Les fistules peuvent être maintenues actives pendant de très nombreuses années, et la peau de certains dialysés est peu souple, mince et fragile du fait de l'âge.

Dans une première étape, représentée en figure 2, l'aiguille 3 est retirée et une bande adhésive 4 est placée au point de ponction. Cette bande adhésive 4 est transparente, semi-transparente ou translucide.

Avantageusement, cette bande adhésive 4 est perméable aux liquides, par exemple microperforée.

Avantageusement, cette bande adhésive 4 est porteuse de composés hémostatiques, par exemple mélangés à l'adhésif.

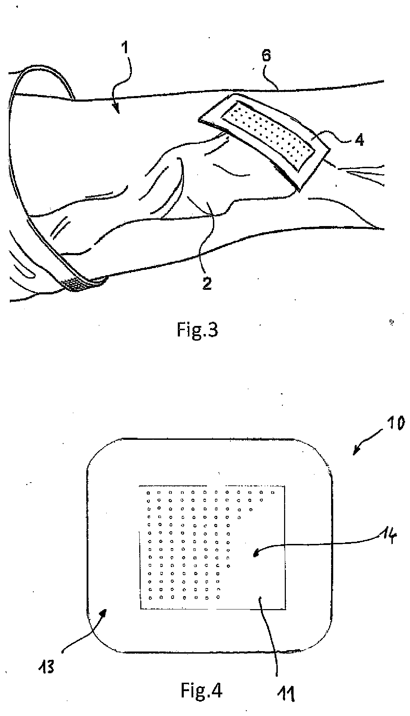

Dans une troisième étape, représentée en figure 3, une compresse peut être appliquée avec pression contre la bande adhésive 4, au droit du point de ponction, l'aiguille 3 étant complètement retirée.

La pression sur la compresse peut ensuite être relâchée et un contrôle visuel de l'hémostase peut est effectué. Il est à noter que la bande adhésive 4 n'a pas à être enlevée, de sorte que le point de ponction reste recouvert lors du contrôle visuel de l'hémostase. Les risques d'AES sont ainsi supprimés, de même que les risques de saignement liés à un enlèvement du clou plaquettaire.

Une bande adhésive, par exemple de type commercialisé sous la marque Tegaderm®, peut ensuite être rapportée au-dessus de la compresse, ou au-dessus de la bande adhésive 4.

La mise en oeuvre de ce procédé permet de réduire fortement le temps de compression aux points de ponction des FAV.

Il résulte de l'expérience du demandeur que les mécanismes conduisant à cette très importante réduction du temps de compression pourraient être les suivants.

Dans une première phase, après mise en place de la bande adhésive recouvrant le point de ponction, le sang sortant du point de ponction ne s'écoule pas sous la bande adhésive 4 et reste confiné. Cette phase de confinement est d'une durée variable, en fonction de l'état d'hydratation du sang total : plus le patient est en hyper hydratation, plus cette première phase est longue. La durée de cette première phase est augmentée par la prise d'anti vitamine K ou en présence d'anomalie plaquettaire. La durée de cette première phase est également augmentée lorsque la FAV est le siège d'une sténose ou lorsque la ponction de la FAV est réalisée sur une zone anévrysmale ou proche de l'anastomose. La durée de cette première phase peut être réduite, en exerçant une pression douce sur l'anastomose de deux minutes environ.

Dans une deuxième phase, plus courte que la première, le sang est tamisé par son passage au travers de la bande adhésive 4, perméable au liquide, et le sérum est absorbé par la compresse. La concentration en éléments figurés augmente dans le sang présent au point de ponction, du fait de ce tamisage et de cette absorption du sérum. La viscosité du sang présent au point de ponction augmente. Les perforations de la bande adhésive 4 sont progressivement obstruées par du sang visqueux.

La mise en oeuvre du procédé permet également de réduire fortement les risques de reprise de saignement.

Il résulte de l'expérience du demandeur que les mécanismes conduisant à cette réduction du risque de reprise de saignement pourraient être les suivants.

Le confinement et le tamisage du sang s'écoulant du point de ponction conduit à la création de croutes de petites étendues. Lors du retrait de la compresse, le risque de décollement de ces croûtes est ainsi réduit.

La bande adhésive 4 semi perméable transparente stérile protège le site de ponction vasculaire et le laisse visible à tout moment sans risque de projection de sang ni de collage direct de la compresse sur le point de ponction.

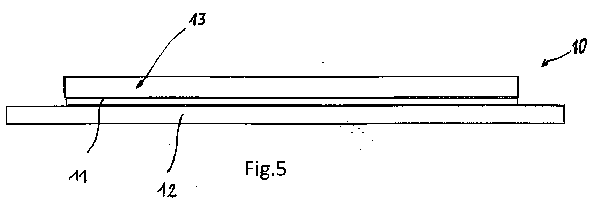

On se reporte maintenant aux figures 4 et 5 illustrant un mode de réalisation d'un pansement, permettant l'emploi du procédé décrit en référence aux figures 1 à 3.

Le pansement 10 représentée en figures 4 et 5 comprend une première partie 11 formant ladite bande adhésive 4. Avantageusement, une feuille pelable 12 vient recouvrir la face adhésive de cette première partie 11. Dans une mise en oeuvre la feuille pelable 12 est à base de polyester siliconé, de faible épaisseur, par exemple de l'ordre de 0.05 mm.

Avantageusement, la bande adhésive 4 est semi-perméable et autorise la diffusion d'au moins certains composés du sang, du point de ponction vers une compresse placée en regard de la bande adhésive 4.

Dans un mode de mise en oeuvre avantageux, la bande adhésive est réalisée en matériau polymère souple, ou tissu enduit, et est avantageusement entièrement transparente, translucide ou semi transparente.

La souplesse du pansement permet de suivre les contours de la peau, y compris au voisinage des zones anévrismales des FAV. Avantageusement, le matériau formant la bande adhésive et le cas échéant tout le pansement 10, est extensible suivant au moins une direction longitudinale et, encore plus avantageusement, suivant les deux directions longitudinale et transversale. Le pansement 10 est ainsi encore plus adaptable aux différentes courbures du corps du patient.

La perforation de la bande adhésive 4 est avantageusement réalisée en mettant en oeuvre un procédé comprenant les étapes suivantes :
- dépôt de colle sur la face du film devant être rendue adhésive, par exemple un film de polyéthylène (PE), notamment un film PE basse densité de 60 microns d'épaisseur, l'adhésif étant à base acrylique, sur une épaisseur de 30 microns environ ;
- dépôt d'un liner recouvrant la face encollée ;
- perforation du film, par exemple par aiguilletage à chaud, suivant un motif à maille carrée ou losange, en une ou deux passes, la densité de trous étant avantageusement de l'ordre de 110 par centimètre carré ;
- dépose du film de protection et mise en place d'une bande pelable.

Avantageusement, les perforations destinées au tamisage sont des micro-perforations obtenues sans enlèvement de matière, par exemple par aiguilletage.

Avantageusement, la bande adhésive 4 est élastique et la perforation est effectuée avec mise sous tension de la bande adhésive, de sorte que les micropores sont sensiblement fermés après relâchement de la tension de la bande. Lors de la pose du pansement, l'étirement manuel de la bande adhésive 4 provoque l'ouverture de ces micropores.

Le pansement comprend un cadre 13 en matériau souple. Le cadre peut être de forme carrée, rectangulaire ou ovale. Avantageusement, le cadre 13 est en mousse, par exemple en mousse de polyéthylène. Dans d'autres mises en oeuvre, le cadre 13 est en matériau non-tissé à base de cellulose, ou est à base de polyéthylène vinyl acétate.

Avant son retrait, l'aiguille occupe un espace entre la peau et la bande adhésive 4, cet espace pouvant former un court chemin de passage au sang sortant du point de ponction. Le présent inventeur a constaté que pour supprimer ce risque, il est avantageux d'appliquer une pression manuelle sur le cadre 13, au-dessus de l'aiguille lors de son retrait.

Avantageusement, le cadre 13 et la bande adhésive 4 ne comportent pas de latex, évitant les réactions allergiques à ce composé.

A titre indicatif, le pansement 10 est, lorsque vu en plan, de contour sensiblement rectangulaire à bords arrondis, de longueur égale à environ 40 mm et de largueur égale à environ 35 mm. Le cadre 13 délimite une fenêtre centrale 14 sensiblement carrée, de 20 mm de côté environ. L'épaisseur du cadre 13 est de l'ordre de 1.5 à 2 mm.

Le pansement 10 qui vient d'être décrit présente de nombreux avantages.

Le présent inventeur a constaté que les risques d'accident par exposition au sang étaient fortement réduits, lors du débranchement des lignes veineuse et artérielle en fin de séance de dialyse.

En effet, chez la plupart des patients, seule une goutte de sang apparaît et perle au point de passage de l'aiguille, cette très faible quantité de sang étant absorbée par une compresse plaquée au-dessus du pansement, le temps de compression étant de quelques dizaines de secondes. Pour quelques rares patients, par exemple en cas de sténose de la FAV ou d'anomalie du rythme cardiaque, l'écoulement du sang peut intervenir par un léger jet, mais cet écoulement est brisé par la présence de la bande adhésive micro-perforée. Le pansement permet un contrôle visuel d'un éventuel saignement prolongé, sans risque de projection.

Le présent inventeur a constaté que les temps de compression en fin de séance de dialyse étaient fortement réduits.

Le cadre 13 peut également participer à une protection du point de ponction ou d'infusion contre la pression et les chocs, maintenant un espace entre la peau et les vêtements du patient. Le cas échéant, une compresse peut être maintenue en place dans la fenêtre 14 définie par le cadre 13.

## Revendications

1. Pansement (10) pour point de ponction ou d'infusion, comprenant une feuille en matériau polymère micro-perforée, une première face de cette feuille étant recouverte d'un adhésif sensible à la pression, la face opposée de cette feuille étant non adhésive, **caractérisé en ce qu'**il comprend une bande de matériau, fixée sur la face non adhésive de la feuille de matériau polymère, cette bande de matériau étant compressible et imperméable, la feuille en matériau polymère micro-perforée est pourvue de perforations d'un diamètre inférieur à 0.5 mm.

2. Pansement (10) pour point de ponction ou d'infusion selon la revendication 1, **caractérisé en ce que** la bande de matériau compressible et imperméable forme un cadre (13).

3. Pansement (10) pour point de ponction ou d'infusion selon la revendication 1 ou 2, **caractérisée en ce que** la bande de matériau compressible et imperméable est en polymère expansé, ou en matériau non-tissé à base de cellulose, ou à base de polyéthylène vinyl acétate.

4. Pansement (10) pour point de ponction ou d'infusion selon la revendication 3, **caractérisé en ce que** la bande de matériau compressible et imperméable est en mousse de polyéthylène.

5. Pansement (10) pour point de ponction ou d'infusion selon l'une quelconque des revendications 1 à 4, **caractérisé en ce que** la feuille en matériau polymère micro-perforée est transparente, translucide ou semi-transparente.

6. Pansement (10) pour point de ponction ou d'infusion selon la revendication 5, **caractérisé en ce que** la feuille en matériau polymère micro-perforée est en polyéthylène pourvue d'un adhésif acrylique.

7. Pansement (10) pour point de ponction ou d'infusion selon la revendication 6, **caractérisé en ce que** la densité de perforations de la feuille en matériau polymère micro-perforée est de l'ordre de cent par centimètre carré.

8. Kit de perfusion, de drainage, ou de cathétérisme, comprenant un pansement (10) tel que présenté dans l'une quelconque des revendications précédentes, et une aiguille ou une canule ou un cathéter ou un drain.

## Patentansprüche

1. Verbandmaterial (10) für Punktions- oder Infusionsstelle, umfassend einen mikroperforierten Bogen aus Polymermaterial, wobei eine erste Fläche dieses Bogens mit einem drucksensiblen Haftstoff bedeckt ist, wobei die gegenüberliegende Fläche dieses Bogens nicht haftend ist, **dadurch gekennzeichnet, dass** es einen Materialstreifen umfasst, der auf der nicht haftenden Fläche des Polymermaterialbogens befestigt ist, wobei dieser Materialstreifen komprimierbar und feuchtigkeitsbeständig ist, wobei der mikroperforierte Bogen aus Polymermaterial mit Perforationen mit einem Durchmesser von unter 0,05 mm versehen ist.

2. Verbandmaterial (10) für Punktions- oder Infusionsstelle nach Anspruch 1, **dadurch gekennzeichnet, dass** der komprimierbare und feuchtigkeitsbeständige Materialstreifen einen Rahmen (13) bildet.

3. Verbandmaterial (10) für Punktions- oder Infusionsstelle nach Anspruch 1 oder 2, **dadurch gekennzeichnet, dass** der komprimierbare und feuchtigkeitsbeständige Materialstreifen aus expandiertem Polymer oder aus Vliesstoffmaterial auf Zellulosebasis oder auf der Basis von Polyethylenvinylacetat ist.

4. Verbandmaterial (10) für Punktions- oder Infusionsstelle nach Anspruch 3, **dadurch gekennzeichnet, dass** der komprimierbare und feuchtigkeitsbeständige Materialstreifen aus Polyethylenschaumstoff ist.

5. Verbandmaterial (10) für Punktions- oder Infusionsstelle nach einem der Ansprüche 1 bis 4, **dadurch gekennzeichnet, dass** der mikroperforierte Bogen aus Polymermaterial transparent, durchscheinend oder halbtransparent ist.

6. Verbandmaterial (10) für Punktions- oder Infusionsstelle nach Anspruch 5, **dadurch gekennzeichnet, dass** der mikroperforierte Bogen aus Polymermaterial aus Polyethylen, versehen mit einem Acrylhaftstoff, ist.

7. Verbandmaterial (10) für Punktions- oder Infusionsstelle nach Anspruch 7, **dadurch gekennzeichnet, dass** die Dichte der Perforationen des mikroperforierten Bogens aus Polymermaterial zirka einhundert je Quadratzentimeter beträgt.

8. Perfusions-, Drainage- oder Katheterset, umfassend ein Verbandmaterial (10) nach einem der vorangehenden Ansprüche und eine Nadel oder eine Kanüle oder einen Katheter oder einen Drain.

## Claims

1. A dressing (10) for a puncture or infusion site, comprising a sheet of micro-perforated polymeric material, a first face of this sheet being covered with a pressure-sensitive adhesive, the opposite face of this sheet being non-adhesive, **characterised in that** it comprises a strip of material, attached to the non-adhesive face of the sheet of polymeric material, this strip of material being compressible and impervious, the sheet of micro-perforated polymeric material is provided with perforations having a diameter lower than 0.5mm.

2. The dressing (10) for a puncture or infusion site according to claim 1, **characterised in that** the compressible and impervious strip of material forms a frame (13).

3. The dressing (10) for a puncture or infusion site according to claim 1 or 2, **characterised in that** the compressible and impervious strip of material is of expanded polymer, or of a cellulose based, or polyethylene vinyl acetate based nonwoven material.

4. The dressing (10) for a puncture or infusion site according to claim 3, **characterised in that** the compressible and impervious strip of material is of polyethylene foam.

5. The dressing (10) for a puncture or infusion site according to any of claims 1 to 4, **characterised in that** the sheet of micro-perforated polymeric material is transparent, translucent or semi-transparent.

6. The dressing (10) for a puncture or infusion site according to claim 5, **characterised in that** the sheet of micro-perforated polymeric material is of polyethylene provided with an acrylic adhesive.

7. The dressing (10) for a puncture or infusion site according to claim 6, **characterised in that** the perforation density of the sheet of micro-perforated polymeric material is in the order of one hundred per square centimetre.

8. A perfusion, drainage, or catheterism kit, comprising a dressing (10) such as set forth in any of the previous claims, and a needle or a canula or a catheter or a drain.
